# EUROPÄISCHE PATENTSCHRIFT

(11) **EP 2 415 356 B1**
(45) Veröffentlichungstag und Bekanntmachung des Hinweises auf die Patenterteilung: **22.05.2013**
(21) Anmeldenummer: 11004990.5
(22) Anmeldetag: 18.06.2011
(51) Int. Cl.: A23K 1/00, A23K 1/18

(54) **Mikroverkapseltes Nahrungssubstrat zur Zucht von räuberischen Spinnentieren**
Microcapsule nutrition substrate for breeding predatory spiders
Substrat d'alimentation microencaspulé pour la culture d'araignées prédatrices

(30) Priorität: 02.08.2010 DE 102010033048
(43) Veröffentlichungstag der Anmeldung: 08.02.2012
(73) Patentinhaber: Katz Biotech AG, 15837 Baruth/Mark (DE)
(72) Erfinder: Katz, Peter, Dr., 12203 Berlin (DE); Rademacher, Jörg, 12277 Berlin (DE); Pommersheim, Rainer, Dr., 55118 Mainz (DE)
(74) Vertreter: Sacht-Gorny, Gudrun

(56) Entgegenhaltungen:
- EP-A1- 0 244 118
- WO-A1-01/30144
- WO-A1-90/00005
- WO-A1-2006/032019
- WO-A2-03/000047
- US-A- 6 004 571
- Rainer POMMERSHEIM et al.: "Immobilization of enzymes by multilayer microcapsules", MACROMOLECULAR CHEMISTRY AND PHYSICS, Bd. 195 1. Januar 1994 (1994-01-01), Seiten 1557-1567, XP55014643, Gefunden im Internet: URL:http://onlinelibrary.wiley.com/store/1 0.1002/macp.1994.021950508/asset/021950508 _ftp.pdf?v=1&t=gw4p4rdr&s=84eb362965d50409 822db1129e561fca18a44f7a [gefunden am 2011-12-13]
- SHI X-Y ET AL: "Preparation of chitosan/ethylcellulose complex microcapsule and its application in controlled release of Vitamin D2", BIOMATERIALS, ELSEVIER SCIENCE PUBLISHERS BV., BARKING, GB, Bd. 23, Nr. 23, 1. Dezember 2002 (2002-12-01), Seiten 4469-4473, XP004377517, ISSN: 0142-9612, DOI: 10.1016/S0142-9612(02)00165-5

## Beschreibung

Die vorliegende Erfindung betrifft die Verwendung eines mikroverkapselten Nahrungssubstrats, eine Zusammensetzung und ein Verfahren zur Zucht von räuberischen Spinnentieren, ein Präparat zur biologischen Schädlingsbekämpfung sowie ein Verfahren zur Herstellung von Mikrokapseln.

Die biologische Schädlingsbekämpfung ist in landwirtschaftlichen und gartenbaulichen Kulturen weit verbreitet. So werden bereits Raubmilben eingesetzt, um beispielsweise verschiedene pflanzenschädigende Insekten und Spinnmilben zu bekämpfen. Wichtige Vertreter der im biologischen Pflanzenschutz eingesetzten Raubmilbenarten sind mehrere Arten der Familie der Phytoseiidae, beispielsweise *Phytoseiulus persimilis, Amblyseius cucumeris, Amblyseius swirskii* und *Typhlodromus pyri,* sowie Arten der Familie der Laelapidae, beispielsweise *Hypoaspis miles.* Diese verschiedenen Arten unterscheiden sich in Bezug auf ihren Zielorganismus, also auf den Pflanzenschädling, der durch sie bekämpft werden kann. *Amblyseius swirskii* ist beispielsweise zur Bekämpfung der Weißen Fliege, von Thripsen und von Spinnmilben geeignet. *Typhlodromus pyri* kann gegen Spinnmilben allgemein eingesetzt werden, während *Phytoseiulus persimilis* speziell zur Bekämpfung der Gemeinen Spinnmilbe einsetzbar ist.

Bei Raubmilben, die ein breites Beutespektrum zeigen, spricht man von polyphagen Räubern (z.B. *Amblyseius swirskii*). Oligophage Arten (z.B. *Typhlodromus pyri*) ernähren sich von nur wenigen Arten oder akzeptieren andere Ersatznahrung, während monophage Räuber (z.B. *Phytoseiulus persimilis*) sich nur von einer Art ernähren können.

Um Raubmilben für die biologische Schädlingsbekämpfung einsetzen zu können, müssen die Raubmilben in geeigneter Menge bereitgestellt werden, um sie dann in die Pflanzenkultur einbringen zu können. Für die Produktion der Raubmilben wird in der Regel eine Massenzucht durchgeführt, wobei als Futtermittel für die Raubmilben bzw. die Nützlinge verschiedene Beuteorganismen als Lebendfutter eingesetzt werden. Als Beuteorganismus kann beispielsweise die natürliche Beute der Raubmilbe gewählt werden. Bei oligo- und polyphagen Arten ist es auch im Allgemeinen möglich, statt der natürlichen Beute eine Ersatzbeute einzusetzen.

Die Zielorganismen, also die natürliche Beute der Raubmilben, sind die jeweiligen Pflanzenschädlinge, die bekämpft werden sollen. Für die Massenzucht der Raubmilben müssen diese Pflanzenschädlinge zunächst auf Pflanzen vermehrt werden, bevor sie als Nahrung für die Raubmilben dienen können. Dies erfordert eine Produktion der Pflanzen, die üblicherweise im Zuge einer sehr flächen- und energieintensiven Pflanzenproduktion in Gewächshausanlagen erfolgt. Auf diesen Pflanzen erfolgt dann die Vermehrung der Pflanzenschädlinge, bevor dann die Pflanzenschädlinge zur Zucht der Raubmilben eingesetzt werden können. Dieses Verfahren ist sehr aufwändig und wird im Allgemeinen nur für solche Raubmilben eingesetzt, die sich ausschließlich von dem natürlichen Zielorganismus ernähren können (monophag) oder für die keine geeignete Ersatzbeute zur Verfügung steht.

Durch die Verwendung von Ersatzbeute als Lebendfutter für die zu züchtenden Raubmilben kann das Massenzuchtverfahren wesentlich vereinfacht werden. Im Allgemeinen werden als Ersatzbeute Milben verwendet, die beispielsweise in Vorratslagern als Schädlinge auftreten und die von der jeweiligen Raubmilbe als Ersatzbeute akzeptiert wird. Die Massenzucht mit einer Ersatzbeute gelang zunächst bei der Raubmilbe *Amblyseius cucumeris,* die mit Modermilben (*Tyrophagus sp*.), die zu der Ordnung Astigmata zählen, gefüttert werden kann. Modermilben können auf entsprechendem Substrat in großen Massen vermehrt werden, sodass hierdurch das Massenzuchtverfahren für die Raubmilben durch den Verzicht auf eine Pflanzenproduktion für den Beuteorganismus erheblich vereinfacht werden konnte.

Für die Zucht der polyphagen Raubmilbe *Amblyseius swirskii* können als Ersatzbeute bzw. als Futtermilben Dörrobstmilben (*Carpoglyphus lactis*) eingesetzt werden, die sich in einer Mischung aus Kleie und einer stark zuckerhaltigen Diät vermehren lassen. So beschreibt beispielsweise die internationale Patentanmeldung WO 2006/057552 A1 die Verwendung einer Astigmatid-Milbe aus der Familie der *Carpoglyphidae* zur Zucht der Raubmilbe *Amblyseius swirskii.* Die internationale Patentanmeldung WO 2007/075081 A1 beschreibt die Zucht von Phytoseiden-Raubmilben, beispielsweise auch *Amblyseius swirskii,* unter Verwendung einer Futtermilbe aus der Familie der *Glycyphagidae.* Die internationale Patentanmeldung WO 2008/104807 A2 beschreibt ein Zuchtverfahren für Raubmilben, beispielsweise *Amblyseius swirskii,* unter Verwendung von Futtermilben aus den Familien *Suidasiidae* oder *Chortoglyphidae.*

Für eine erfolgreiche Vermehrung der Raubmilben sind im Allgemeinen circa 100 Futtermilben pro Raubmilbe erforderlich. Diese hohe Anzahl von Beuteorganismen ist nötig, da die Raubmilben oftmals eine besondere Präferenz für beispielsweise die Eier der Futtermilben zeigen. Die sehr hohe Individuendichte der Futtermilben führt dazu, dass sich schädliche Stoffwechselgase und andere Ausscheidungen schnell anreichern können. Die Schichtdicke und die Individuendichte auf dem Zuchtsubstrat sind daher begrenzt, da eine ausreichende Ventilation sichergestellt werden muss.

Gegenüber der Verwendung der natürlichen Ziel- bzw. Beuteorganismen hat der Einsatz von einer Ersatzbeute für die Zucht zwar den Vorteil, dass keine sehr flächenund arbeitsaufwandintensive Pflanzenproduktion mehr erforderlich ist. Jedoch auch bei der Verwendung von Ersatzbeute entfallen auf die Vermehrung der Futtermilben etwa noch 2/3 des gesamten Aufwandes in Bezug auf die Produktionsfläche und die Arbeitszeit.

Ein weiterer Nachteil bei der Verwendung von Ersatzbeute bei der Massenzucht von Raubmilben ergibt sich aus der direkten Wirkung der Futtermilben auf die Umwelt. Zunächst können die Ausscheidungen und/oder die Futtermilben selbst beim Anwender und bei den Produktionsmitarbeitern allergische Reaktionen auslösen. Weiterhin sind Schäden an den Kulturpflanzen selbst durch die Futtermilben nicht auszuschließen, da bei dem Ausbringen der Raubmilben in den entsprechenden Präparaten in der Regel auch die Futtermilben selbst in großer Anzahl enthalten sind. Auch Futtermilben, die primär keine Pflanzenschädlinge sind, können unter ungünstigen klimatischen Bedingungen Pflanzenschäden verursachen, wenn sie zusammen mit den Raubmilben in die Pflanzenkultur eingebracht werden. Weiterhin besteht bei einer lang andauernden Massenzucht auf der Basis von Ersatzbeute die Gefahr, dass die Raubmilben mit der Zeit die Fähigkeit zur Erkennung und Erbeutung ihrer eigentlichen Zielorganismen reduzieren, sodass sie bei dem Einsatz in der Schädlingsbekämpfung weniger effektiv sind.

Im Allgemeinen ist es sehr schwierig, für eine bestimmte Raubmilbe einen geeigneten Beuteorganismus als Ersatzbeute zu finden. Eine geeignete Ersatzbeute muss neben der Akzeptanz durch die Raubmilbe auch weiterhin für eine Massenvermehrung unter annehmbaren Bedingungen geeignet sein. Weiterhin müssen die Kulturbedingungen für die Raubmilbe beispielsweise im Hinblick auf Temperatur, Feuchtigkeit usw. in Übereinstimmung mit den Kulturbedingungen für die Futtermilbe gebracht werden können, da sowohl die Raubmilbenpopulation als auch die Futtermilbenpopulation sich parallel während der Massenzucht in einer Kultur vermehren lassen sollten. Zudem muss das Risiko bedacht werden, dass durch die Massenvermehrung und die Einbringung der Futtermilben auch in die Pflanzenkultur mit Wirkungen auf den Menschen, die Umwelt und auch auf die Pflanzen zu rechnen ist. Schließlich besteht die Gefahr, dass bei der herkömmlichen Verwendung von Lebendfutter für die Zucht der räuberischen Spinnentiere diese mit der Zeit die Fähigkeit zur Erkennung ihrer eigentlichen Zielorganismen reduzieren, sodass sie bei dem Einsatz in der Schädlingsbekämpfung weniger effektiv sind.

Das Auffinden einer geeigneten Futtermilbe als Ersatzbeute für Raubmilben ist insbesondere mit zunehmender Beutespezialisierung der Raubmilben sehr schwierig. Während bei der polyphagen Raubmilbe *Amblyseius swirskii* beispielsweise noch verschiedene Ersatzbeuteorganismen akzeptiert werden, kann sich die monophage Raubmilbe *Phytoseiulus persimilis* nur von der Gemeinen Spinnmilbe *Tetranychus urticae* ernähren. Die Massenzucht der Raubmilbe *Phytoseiulus persimilis* ist aufgrund der Nahrungsspezialisierung nur auf der Basis des natürlichen Zielorganismus möglich und nur in begrenztem Umfang durchführbar. Ein wirtschaftlicher Einsatz der Raubmilbe *Phytoseiulus persimilis* insbesondere auch in Freilandkulturen ist daher oft nicht möglich.

Es sind bereits verschiedene Ansätze beschrieben worden, um Raubmilben unter Verwendung eines künstlichen Substrates zu züchten. So beschreibt die internationale Patentanmeldung WO 99/59402 A1 ein künstliches Medium zur Zucht von Milben, Insekten und anderen nützlichen Organismen, dass eine Fettquelle, eine Aminosäurequelle, wenigstens ein Protein und eine Kohlehydratquelle aufweist. Dieses Substrat kann in Form von Alginat-Kügelchen oder Carboxymethylcellulose-Kügelchen bereitgestellt werden.

Die US-Patentanmeldung US 2002/0050659 A1 beschreibt ein Verfahren zur Herstellung von Mikrokapseln mit einem flüssigen Futtermittel für z.B. die Zucht von Insekten, bei dem ein flüssiges Material für den Inhalt und ein polymerisierbares Material für die Hülle simultan extrudiert werden. Das polymerisierbare Material besteht aus funktionalisierten Präpolymeren wie z.B. Silikon, Urethan, Polyepoxiden, Polyester oder Vinylmonomeren, die mit Hilfe eines Energieeintrags, z.B. UV-Strahlung, quervernetzt werden.

Auch die US-Patentschrift US 5,799,607 beschreibt ein künstliches Medium zur Zucht von Insekten, das durch die Verwendung von polymeren Filmbildnern wie z.B. Polyethylen, Polypropylen, Polyvinylchlorid oder Parafilm verkapselt wird. Hierbei wird das Medium in Folien eingeschweißt, die aus diesen Materialien gebildet sind.

Die US-Patentschrift US 6,004,571 beschreibt ein künstliches Insektenei, das aus einem Tropfen Nährlösung besteht, der von einer polymerisierten Schale umgeben ist. Für die Herstellung der Schale wird ein Quervernetzungsmittel, z.B. Säurechlorid oder Isocyanat, verwendet, das die Quervemetzung von Protein, die aus der Nährlösung selbst stammen oder zugesetzt wurden, verwendet. Diese Kapsel wird mit einer Wachsschicht umgeben, indem die einzelnen Kapseln mit einer Pinzette in ein Wachsbad getaucht werden. Dieses Herstellungsverfahren ist nicht für eine Massenproduktion des Substrats geeignet.

Die Veröffentlichung von Pommersheim et al. in Macromol. Chem. Phys., Bd. 195 (1994), Seiten 1557 - 1567 beschreibt ein Verfahren zur Herstellung von Mikrokapseln mit einer semipermeablen Membran zur Verkapselung von z.B. Enzymen oder Zellen, deren Freisetzung beeinflusst werden soll. Hierbei werden Hüllschichten aus Polyethylenimin und Polyacrylamid aufgebaut, die für die aktiven Substanzen oder Partikel permeabel sind.
Die Veröffentlichung WO 01/30144 A1 einer internationalen Patentanmeldung beschreibt die Herstellung verschiedener Mikrokapseln, deren Hüllschichten die Diffusion der Inhaltsstoffe der Kapseln kontrollieren sollen.

Die mit diesen verschiedenen Methoden herstellbaren Mikrokapseln haben alle den Nachteil, dass die Massenzucht von räuberischen Spinnentieren unter Verwendung derartiger Mikrokapseln nicht erfolgreich ist. Insbesondere werden die Mikrokapseln von den räuberischen Spinnentieren aus verschiedenen Gründen nicht als Futtermittel akzeptiert, sodass keine befriedigenden Vermehrungsraten erzielt werden, und/oder die Mikrokapseln sind unter den Massenzuchtbedingungen nicht ausreichend stabil. Dies hat dazu geführt, dass sich keiner der beschriebenen Ansätze mit synthetischem Futtersubstrat in der Praxis durchgesetzt hat.

Der Erfindung liegt demgegenüber die Aufgabe zugrunde, die Massenzucht von Raubmilben und allgemein von räuberischen Spinnentieren zur biologischen Schädlingsbekämpfung zu verbessern. Die Nachteile, die mit der Verwendung von Ersatzbeute bei der Massenzucht einhergehen, sollen vermieden werden. Insbesondere soll das Schadensrisiko bei der Verwendung von Futtermilben im Hinblick auf ihre Wirkungen auf Menschen und Umwelt minimiert werden. Weiterhin soll auch die Zucht von Raubmilben, die ein sehr spezifisches Beutespektrum zeigen, vereinfacht und eine Massenzucht gegebenenfalls erst ermöglicht werden. Durch die Entwicklung von Mikrokapseln, die an die speziellen Bedürfnisse der räuberischen Spinnentiere angepasst sind, soll eine Zucht von Raubmilben oder anderen Spinnentieren in größerem Maßstab ermöglicht werden, sodass diese nützlichen Organismen für die biologische Schädlingsbekämpfung in der erforderlichen Menge bereitgestellt werden können.

Diese Aufgabe wird durch die Verwendung eines mikroverkapselten Nahrungssubstrats gelöst, wie es Gegenstand des Anspruchs 1 ist. Bevorzugte Ausgestaltungen dieser Verwendung sind Gegenstand der abhängigen Ansprüche. Weiterhin wird die Aufgabe durch eine Zusammensetzung und ein Verfahren für die Massenzucht von räuberischen Spinnentieren, durch ein Präparat zur biologischen Schädlingsbekämpfung sowie durch eine Verfahren zur Herstellung von Mikrokapseln und entsprechend herstellbare Mikrokapseln gelöst, wie es sich aus den weiteren unabhängigen Ansprüchen ergibt.

Die Erfindung sieht die Verwendung eines mikroverkapselten Nahrungssubstrats für die Massenzucht von räuberischen Spinnentieren vor. Hierdurch wird die Grundlage für den Aufbau einer hocheffizienten Massenzucht von räuberischen Spinnentieren wie beispielsweise Raubmilben bereitgestellt, sodass beispielsweise die Raubmilben zur effektiven biologischen Schädlingsbekämpfung in landwirtschaftlichen und gartenbaulichen Kulturanpflanzungen eingesetzt werden können. Die bisherigen Ansätze zur Massenzucht von Raubmilben basieren größtenteils auf der Verwendung von Lebendfutter, entweder auf der Verwendung des natürlichen Beuteorganismus oder, sofern möglich, von Ersatzbeute. In jedem Fall ist es bei diesen herkömmlichen Verfahren erforderlich, den Beuteorganismus ebenfalls in ausreichender Menge zu züchten. Dies ist in den allermeisten Fällen mit erheblichem Aufwand verbunden, wie eingangs beschrieben.

Das erfindungsgemäß verwendete Nahrungssubstrat umfasst Mikrokapseln, die eine feste Hülle aus im Wesentlichen natürlichen Polymeren aufweisen. Erfindungsgemäß umfasst die feste Hülle eine Diffusionssperrschicht. Die Diffusionssperrschicht umfasst Ethylcellulose und/oder Polymere auf Harzbasis, insbesondere Schellack. Die Diffusionssperrschicht der Hülle verhindert oder verzögert eine Verdunstung des Lösungsmittels aus dem Inneren der Mikrokapsel, sodass die Haltbarkeit der Mikrokapseln über einen längeren Zeitraum gewährleistet ist. Dies ist eine Voraussetzung für eine erfolgreiche Verwendung der Mikrokapseln für die Massenzucht der räuberischen Spinnentiere.

Das im Inneren der Mikrokapseln vorliegende Futtermittel ist vorzugsweise flüssig, sodass die eigentliche Nahrung für die zu züchtenden Spinnentiere in flüssiger Form vorliegt, insbesondere als wässrige Lösung. Die Viskosität des flüssigen Futtermittels kann über der Viskosität von beispielsweise Wasser liegen und sich beispielsweise im Bereich zwischen 100 bis zu 1000 mPa·s bewegen. Vorzugsweise sollte das Futtermittel in gießbarer Form vorliegen. Die umgebende Hülle, die die Diffusionssperrschicht umfasst, weist eine höhere Viskosität und Dichte auf, sodass das Innere der Mikrokapsel in Relation hierzu eine niedrige Viskosität aufweist. Durch die relativ niedrige Viskosität im Inneren der Mikrokapseln sind die räuberischen Spinnentiere mit Hilfe ihrer saugenden Mundwerkzeuge in der Lage, die in dieser Lösung vorliegenden Nahrungspartikel aufzunehmen.

Wichtig für die Erfindung ist weiterhin, dass die feste Hülle natürliche Polymere umfasst, das heißt, dass die feste Hülle im Wesentlichen von natürlichen Polymeren gebildet wird. Hierbei sind unter dem Begriff "natürliche Polymere" solche Polymere zu verstehen, die einen natürlichen, d.h. biologischen Ursprung haben oder von natürlich vorkommenden Polymeren abgeleitet sind, wie beispielsweise Derivate. Insgesamt umfasst der Begriff "natürliche Polymere" also auch naturähnliche Polymere. Die erfindungsgemäß eingesetzten natürlichen Polymere (Biopolymere) für die Ausbildung der Hülle bewirken, dass die Mikrokapseln für die räuberischen Spinnentiere attraktiv sind und als Nahrungsquelle akzeptiert werden. Da insbesondere Raubmilben sehr spezifische Anforderungen an ihre Nahrung stellen, ist der Einsatz von natürlichen Polymeren für den Erfolg der Erfindung wesentlich. Hierbei ähneln die verwendeten Hüllmaterialen vorzugsweise den natürlichen Biopolymeren, die in oder auf den Beuteorganismen der Raubmilben zu finden sind. Die verwendeten Hüllmaterialien üben somit eine attraktive Wirkung auf die Raubmilben oder andere zu züchtende Tiere aus. Weiterhin hat die Verwendung von natürlichen Hüllmaterialen den Vorteil, dass die damit hergestellten Produkte ohne Weiteres auch in Betrieben eingesetzt werden können, die nach ökologischen Richtlinien produzieren.

Allgemein hat der Einsatz von Mikrokapseln als Nahrungssubstrat verschiedene Vorteile. Die Mikrokapseln lassen sich prozesstechnisch sehr gut verarbeiten und erlauben beispielsweise dreidimensionale Zuchtsysteme. Mikrokapseln sind im Prinzip streufähig, sodass der Zuchtansatz beispielsweise von der Fläche in die Höhe ausgedehnt werden kann. Auch der Transport der räuberischen Spinnentiere zusammen mit den Mikrokapseln, die das flüssige Nahrungsmittel enthalten, ist anders als bei einem flüssigen Substrat unproblematisch. Die feste Hülle der Mikrokapseln schützt das flüssige Futtermittel vor schädlichen Umwelteinflüssen, wie Austrocknung, Zerstörung der Nährstoffe durch den Einfluss von UV-Strahlung oder Sauerstoff, Verunreinigung, Krankheitserregern oder zersetzenden Mikroorganismen. Durch die erfindungsgemäße Verwendung eines mikroverkapselten Nahrungssubstrats für die Massenzucht der räuberischen Spinnentiere kann die Massenzucht der Spinnentiere vereinfacht und wirtschaftlich verbessert werden. Das mikroverkapselte Nahrungssubstrat kann in großen Mengen unabhängig von klimatischen und anderen Bedingungen bereitgestellt und gegebenenfalls gelagert werden, sodass es für die Zucht der räuberischen Spinnentiere im Prinzip unbegrenzt und jederzeit zur Verfügung steht. Die erfindungsgemäß verwendeten Mikrokapseln erlauben erstmals eine erfolgreiche Massenzucht von räuberischen Spinnentieren unter Verwendung eines künstlichen Nahrungssubstrats, dass auf den Einsatz von Lebendfutter verzichtet.

Ein Problem bei der herkömmlichen Verwendung von Lebendfutter für die Zucht der räuberischen Spinnentiere ist, dass mit dem Lebendfutter weitere Organismen in die Pflanzenkultur eingebracht werden, deren Auswirkungen auf die Umwelt und die zu schützenden Pflanzen im Allgemeinen schwer abschätzbar sind. Dieses Problem lässt sich bei aus dem Stand der Technik bekannten Zuchtverfahren nicht vermeiden, da es für den Transport der Nützlinge, also beispielsweise der Raubmilben, erforderlich ist, sie ständig mit Nahrung, also mit Lebendfutter, zu versorgen, um Qualitätsverluste während des Transports zum Anwender zu vermeiden. Bei der erfindungsgemäßen Verwendung eines mikroverkapselten Nahrungssubstrats für die Massenzucht tritt dieses Problem nicht auf, da kein Lebendfutter in den Populationen der räuberischen Spinnentiere erforderlich ist. Schließlich wird auch das allergische Risiko, das durch die herkömmlicherweise eingesetzten Futtermilben verursacht wird, für die mit den Präparaten in Kontakt kommenden Personen erheblich reduziert, da im Allgemeinen von dem mikroverkapselten Nahrungssubstrat kein allergisches Risiko ausgeht.

Die Verwendung eines mikroverkapselten Nahrungssubstrats für die Massenzucht von räuberischen Spinnentieren und insbesondere von Raubmilben erlaubt eine hocheffiziente Produktion durch den vereinfachten und sehr gut zu steuernden Produktionsprozess bei reduziertem Flächenbedarf. Die Verwendung des mikroverkapselten Nahrungssubstrats erlaubt eine optimale Nahrungsversorgung der Spinnentiere während der gesamten Produktion und während des Transports zum Anwender und führt damit zu einer deutlichen Qualitätssteigerung. Unvorhersehbare und unerwünschte Nebenwirkungen von herkömmlicherweise eingesetzten Beuteorganismen auf die Pflanzenkultur und die Produktionsmitarbeiter und Anwender treten bei einer Massenzucht gemäß der Erfindung nicht auf.

Die natürlichen Polymere, die erfindungsgemäß Bestandteil der festen Hülle der Mikrokapseln sind, sind vorzugsweise Polysaccharide, insbesondere Chitin, Derivate von Chitin, vorzugsweise Chitosan, Alginat, Carrageen, Pektin, Gummiarabikum und/oder Cellulosederivate wie z.B. Carboxymethylcellulose. Weiterhin sind Proteine wie beispielsweise Gelatine, oder Mischungen der genannten Substanzen geeignet. Vor allem Chitin und/oder Chitosan und Pektin und/oder Carboxymethylcellulose sind als Substanzen für die Hülle jeweils für sich oder in Kombination miteinander bevorzugt, da diese Substanzen in Versuchen eine besonders hohe Attraktivität für Raubmilben gezeigt haben. Vorzugsweise werden für die Ausbildung der festen Hülle wenigstens ein Polykation (z.B. Chitosan) und wenigstens ein Polyanion (z.B. Pektin oder Carboxymethylcellulose) eingesetzt, sodass die Hülle aus Polyelektrolyt-Komplexen aufgebaut wird.

Die feste Hülle der Mikrokapseln kann, gegebenenfalls auch unter Verzicht auf die Polyelektrolyt-Komplexe, ausschließlich aus Naturstoffen gebildet sein, die gute filmbildende Eigenschaften aufweisen und zur Ausbildung der Diffusionssperrschicht geeignet sind. Ein besonders effektiver Verdunstungsschutz lässt sich beispielsweise mit einer Kombination von Ethylcellulose und Schellack erzielen, wobei zugleich eine Attraktivität der Mikrokapseln für die zu züchtenden Spinnentiere erreicht wird. Besonders vorteilhaft ist es, die Diffusionssperrschicht mit einer Hülle aus Polyelektrolyt-Komplexen, wie im vorstehenden Absatz beschrieben, zu kombinieren, da hierdurch die Attraktivität für die zu züchtenden Tiere weiter gesteigert werden kann.

Die Diffusionssperrschicht kann integrierter Bestandteil der festen Hülle sein, oder die Diffusionssperrschicht ist als zusätzliche Schicht auf den Mikrokapseln aufgebracht. Besonders bevorzugt ist es, wenn die Polymere, die die feste Hülle bilden, und die Substanzen, die die Diffusion des Lösungsmittels verhindern, gemeinsam eine verflochtene Struktur bilden, die zum Einen formstabilisierend für die Mikrokapseln ist und zum Anderen den Diffusionsschutz bewirkt. Als formstabilisierende Substanzen können beispielsweise Chitosan, Pektin und/oder Carboxymethylcellulose verwendet werden, wobei als verdunstungsschützende Substanzen Ethylcellulose und/oder Schellack eingesetzt werden. Das Abscheiden der verdunstungsschützenden Substanzen auf der Oberfläche der Mikrokapseln erfolgt vorzugsweise durch Ausfällen, indem das Lösungsmittel verdunstet wird, ohne das eine chemische Quervemetzung der Polymere erforderlich wäre. Die Ausbildung einer Diffusionssperrschicht bei den Mikrokapseln ist vor allem aus prozesstechnischen Gründen bei der Massenzucht der Spinnentiere sehr vorteilhaft, da durch die Diffusionssperrschicht die Mikrokapseln über einen längeren Zeitraum vor Verdunstung geschützt und somit stabil und einsatzfähig, d.h. für die Spinnentiere verwertbar bleiben.

Mit besonderem Vorteil weist die feste Hülle Strukturen und/oder Substanzen auf, die bei den zu züchtenden räuberischen Spinnentieren einen Fressreiz auslösen, oder, anders ausgedrückt, die für die räuberischen Spinnentiere attraktiv sind. Diese Strukturen und/oder Substanzen können auf der Hülle angelagert sein und/oder Bestandteile der Hülle sein. Derartige Strukturen und/oder Substanzen können, je nach Nahrungsspezialisierung des zu züchtenden Tieres die Voraussetzung sein, dass das jeweilige Spinnentier das angebotene Nahrungssubstrat als Futterquelle akzeptiert und es aufnimmt. Bei den Strukturen und/oder Substanzen, die bei den Spinnentieren eine Attraktivität bewirken, kann es sich um charakteristische Strukturen oder Substanzen handeln, die sich beispielsweise auf der Oberfläche der natürlichen Beuteorganismen befinden. Mit Hilfe spezieller Sensoren an den Vorderbeinen und den Mundwerkzeugen der zu züchtenden Spinnentiere werden diese Substanzen oder Strukturen erkannt und darauf geschlossen, dass es sich um ein Beutetier handelt, sodass die feste Hülle der Mikrokapsel mit den Mundwerkzeugen durchdrungen wird und das flüssige Futtermittel aus der Mikrokapsel aufgenommen wird.

Die Strukturen, beispielsweise Oberflächenstrukturen, und/oder Substanzen, die den Fressreiz auslösen bzw. für die Tiere attraktiv sind, können insbesondere von den natürlichen Beuteorganismen abgeleitet oder diesen natürlichen Substanzen nachempfunden sein. Anhand der natürlichen Beuteorganismen können beispielsweise die wirksamen Grundstoffe für die Auslösung des Fressreizes identifiziert und aufgereinigt oder synthetisch bereitgestellt werden. Beispielsweise kann das Material für die feste Hülle aus pulverisierten natürlichen Beuteorganismen, beispielsweise Spinnenmilbenpulver, hergestellt werden. In anderen Ansätzen kann die Attraktivität verschiedener Substanzen in Versuchen mit den zu züchtenden Tieren getestet werden, sodass geeignete, für die Tiere attraktive Substanzen ausgewählt werden können. Die wirksamen Substanzen können mit weiteren Substanzen kombiniert werden, um das Material für die feste Hülle der Mikrokapseln zu bilden.

Das Material für die feste Hülle der Mikrokapseln kann, wie bereits erläutert, aus Bestandteilen der natürlichen Beuteorganismen hergestellt werden. Bei Bedarf können diese Substanzen um weitere Substanzen ergänzt werden, die für die technische Verarbeitung bei der Mikroverkapselung vorteilhaft sind. Weiterhin kann die Herstellung des Hüllmaterials mit solchen Grundstoffen durchgeführt werden, die der natürlichen Hüllstruktur der Beuteorganismen ähneln und die bereits in etablierten Mikroverkapselungsverfahren als Hüllmaterial für Mikrokapseln eingesetzt werden. Diese etablierten Grundstoffe können mit Materialien der natürlichen Beutehülle ergänzt werden, sodass die Auslösung des Fressreizes bei den zu züchtenden Spinnentieren bzw. die Attraktivität der Mikrokapseln für die Spinnentiere gewährleistet ist.

Der Fressreiz kann beispielsweise durch bestimmte chemische Eigenschaften der festen Hülle, wie z.B. Geschmack oder Geruch, sowie durch anatomische Oberflächenstrukturen auf der festen Hülle so gesteuert werden, dass die zu züchtenden Spinnentiere zur Nahrungsaufnahme angeregt werden. Diese Eigenschaften und Strukturen sind vorzugsweise den natürlichen Beuteorganismen der jeweiligen zu züchtenden Spinnentiere nachgebildet.

Vorzugsweise werden die Größe und die Form der natürlichen Beute bei der Ausgestaltung der Mikrokapseln berücksichtigt. Der Fressreiz bei Raubmilben bei natürlichem Substrat wird vermutlich durch eine Kombination einer Chitinhaut mit der Größe und/oder Form der Beute ausgelöst. Durch die Verwendung von Chitin oder Chitinderivaten oder einer anderen für die Tiere attraktiven Substanz für die Mikroverkapselung des flüssigen Futtermittels und der Wahl einer geeigneten Größe der Mikrokapseln, die der natürlichen Beutegröße nachempfunden ist, kann der Fressreiz ausgelöst werden, sodass die zu züchtenden Spinnentiere zur Nahrungsaufnahme angeregt werden. In bevorzugter Weise beträgt der Durchmesser der Mikrokapseln circa 0,1 bis 2 mm, insbesondere circa 0,5 mm.

In einer besonders bevorzugten Ausgestaltung der erfindungsgemäßen Verwendung beträgt die Schichtdicke der festen Hülle der Mikrokapseln im Wesentlichen bis zu circa 20 µm, insbesondere bis zu circa 10 µm. Besonders bevorzugt ist eine Schichtdicke bis zu 5 µm, insbesondere bis zu 1 µm. Diese Schichtdicke erlaubt es zum einen, dass die räuberischen Spinnentiere die feste Hülle der Mikrokapseln mit ihren Mundwerkzeugen ohne Weiteres durchdringen können. Zum anderen ist diese Schichtdicke ausreichend, um das flüssige Futtermittel innerhalb der Mikrokapsel vor schädlichen Umwelteinflüssen zu schützen, beispielsweise vor dem Austrocknen oder vor einer Zersetzung durch Mikroorganismen. Die Festigkeit der Hülle wird ebenfalls vorzugsweise so gestaltet, dass die Hülle von den Mundwerkzeugen der Spinnentiere leicht durchdrungen werden kann. Als Maß für die Festigkeit der Hülle der Mikrokapseln kann beispielsweise die Reißspannung [N/mm²] herangezogen werden, die anhand der für die Zerstörung der Mikrokapsel erforderlichen Kraft ermittelt werden kann. Vorzugsweise wird die Reißspannung der Mikrokapseln so ausgelegt, dass die Reißspannung etwa im Bereich der Reißspannung der natürlichen Beuteorganismen oder besonders bevorzugt darunter liegt.

Bei dem flüssigen Futtermittel handelt es sich vorzugsweise um eine wässrige Lösung der Nahrungssubstanzen bzw. Nährmittel. Bei den Nahrungssubstanzen kann es sich um eine vollsynthetische Mischung handeln. Diese vollsynthetische Mischung erlaubt eine optimale Kombination aller für die Entwicklung der zu züchtenden Spinnentiere benötigten Nährstoffe, Mineralien und Vitamine in ernährungsphysiologisch geeigneter Form. Die Verwendung eines vollsynthetischen Futtermittels erlaubt eine ununterbrochen kontrollierbare und optimale Versorgung der zu züchtenden Spinnentiere mit den notwendigen Substanzen. Anders als bei der herkömmlichen Verwendung von Lebendfutter ist die Nahrungsversorgung der zu züchtenden Spinnentiere keinen Schwankungen, bedingt durch Veränderungen der herkömmlicherweise verwendeten Populationen der Beuteorganismen, unterworfen. Durch die erfindungsgemäße Verwendung eines mikroverkapselten Nahrungssubstrats mit kontrollierter Zusammensetzung der Inhaltsstoffe kann die Entwicklung der Spinnentiere optimiert werden, sodass ein sehr gutes Zuchtergebnis erzielt werden kann.

Gemäß einer weiteren bevorzugten Ausgestaltung der erfindungsgemäßen Verwendung kann das flüssige Futtermittel teilsynthetisch sein. Die Inhaltsstoffe der Mikrokapsel können zum Teil oder nahezu vollständig natürlich vorkommende Substanzen sein. So kann beispielsweise ein Extrakt aus beispielsweise Hefe, aus Insekten und/oder aus Spinnentieren, z. B. Milben, gewonnen werden, das die Grundlage für das flüssige Futtermittel bildet. Weiterhin kann beispielsweise Eigelb, Milch und/oder Honig als Grundlage für das Futtermittel eingesetzt werden.

Das flüssige Futtermittel ist in seiner Zusammensetzung so aufgebaut, dass es die Entwicklung und Vermehrung der Raubmilben ermöglicht. Es enthält insbesondere alle für die Raubmilbenentwicklung essentiellen Bestandteile. Die Konzentration der Nährstoffe, der Wassergehalt und der pH-Wert entsprechen vorzugsweise im Wesentlichen der natürlichen Beute.

Die Wahl der Zusammensetzung des flüssigen Futtermittels und auch der Zusammensetzung, Struktur und Größe der festen Hülle sind abhängig von dem jeweils zu züchtenden räuberischen Spinnentier. Insbesondere bei in ihrer Nahrungswahl sehr spezialisierten Tieren, beispielsweise monophagen Raubmilben, sind die Zusammensetzung und Struktur der Mikrokapseln an das Nahrungsverhalten des jeweiligen Tieres angepasst. Andere, weniger spezialisierte Arten, beispielsweise polyphage Raubmilben, können mit weniger speziellen mikroverkapselten Nahrungssubstraten versorgt werden. Zur Auffindung der geeigneten Zusammensetzung und Struktur der Mikrokapseln für das jeweils zu züchtende Spinnentier kann eine chemische Analyse der natürlichen Beuteorganismen des jeweiligen Spinnentieres durchgeführt werden, um hieraus eine geeignete Zusammensetzung für das mikroverkapselte Nahrungssubstrat abzuleiten. Auch eine Analyse der Struktur, der Festigkeit der Kutikula und der Form und Größe der bevorzugten Beuteorganismen des jeweiligen Spinnentieres kann für die Herstellung eines geeigneten mikroverkapselten Nahrungssubstrats herangezogen werden. Vorzugsweise werden die Zusammensetzung des flüssigen Futtermittels und die Ausgestaltung der festen Hülle so ausgelegt, dass das Nahrungssubstrat wenigstens zwei Wochen gegenüber Umwelteinflüssen stabil ist. Die Stabilität wird vor allem durch die erfindungsgemäß vorgesehene Diffusionssperrschicht erreicht. Insbesondere ist es vorteilhaft, die Qualität der Nährstoffe über einen Zeitraum von mindestens zwei Wochen stabil zu halten. Durch den Einsatz eines über einen längeren Zeitraum stabilen mikroverkapselten Nahrungssubstrats ist es möglich, die zu züchtenden Spinnentiere in einem geschlossenen System unter optimaler Nahrungsversorgung zu vermehren. Ein geschlossenes System hat den Vorteil, dass eine optimale und automatisierte Steuerung der Umweltbedingungen, die für die Entwicklung und Fortpflanzung der zu züchtenden Spinnentiere erforderlich ist, gewährleistet werden kann. Das Fahren der Produktion in einem geschlossenen System erlaubt weitestgehend einen Ausschluss von Krankheitserregern, die negative Auswirkungen auf die Fitness (Vitalität, Fraß- und Vermehrungsleistung, Entwicklungsgeschwindigkeit usw.) der zu züchtenden Spinnentiere ausüben können. Weiterhin wird der Arbeits- und Zeitaufwand für die Produktion erheblich, bis zu 50 %, reduziert. Dies liegt vor allem an dem Wegfall der Produktion der Beuteorganismen und der dafür oftmals erforderlichen Pflanzenproduktion. Weiterhin kann der Flächenbedarf für die Massenproduktion um bis zu 70 % gegenüber herkömmlichen Verfahren reduziert werden.

In einer besonders bevorzugten Ausgestaltung der Mikroverkapselung werden in das Nahrungssubstrat und insbesondere in die Hülle Strukturen eingebracht, die spezifisch für bestimmte Beuteorganismen sind und bei der Beuteerkennung eine Rolle spielen. Diese Strukturen können den Fressreiz für die zu züchtenden Spinnentiere weiter verstärken. Derartige beutespezifische Marker sorgen für eine während der Zucht andauernde spezifische Prägung der zu züchtenden Spinnentiere im Hinblick auf deren Zielorganismus, sodass während der Zucht nicht das Risiko besteht, dass die Spinnentiere ihre Fähigkeit verlieren, den Zielorganismus zu erbeuten. Die Einbringung der beutespezifischen Marker in das Nahrungssubstrat und insbesondere in die Hülle der Mikrokapseln kann beispielsweise durch die Verwendung von Extrakten der Beutetiere (Schaderregerstrukturen) erfolgen, die in das Material für die Hülle eingemischt oder auf die bereits hergestellte Hülle aufgebracht werden.

Vorzugsweise handelt es sich bei den zu züchtenden Spinnentieren um Milben (Acari), insbesondere um Raubmilben, die breite Anwendungsgebiete in der biologischen Schädlingsbekämpfung haben. Hierbei kann es sich allgemein im Hinblick auf die Nahrungsspezialisierung um polyphage, oligophage oder monophage Raubmilben handeln. In besonders vorteilhafter Weise ist die erfindungsgemäße Verwendung des mikroverkapselten Nahrungssubstrats für die Zucht von Raubmilben der Familie der Phytoseiidae (BERLESE, 1916) und Laelapidae (BERLESE, 1892) geeignet, insbesondere von Raubmilben der Gattungen *Amblyseius, Neoseiulus, Hypoaspis, Phytoseiulus* und *Typhlodromus,* beispielsweise für die polyphage Raubmilbe *Amblyseius swirskii,* die monophage Raubmilbe *Phytoseiulus persimilis* oder die oligophage Raubmilbe *Typhlodromus pyri.*

Die folgende Auflistung zeigt beispielhaft wichtige Raubmilbenarten, die im biologischen Pflanzenschutz eingesetzt werden und die in besonders vorteilhafter Weise mit dem erfindungsgemäßen Massenzuchtverfahren produziert werden können. Herkömmliche Zuchtverfahren für diese Raubmilben basieren im Wesentlichen auf dem Einsatz von Lebendfutter für die Raubmilben. Erfindungsgemäß kann auf Lebendfutter verzichtet werden, wodurch die Produktion erheblich verbessert und wirtschaftlicher gestaltet werden kann.

| **Art** | **Ernährungstyp** | **Zielorganismus** |
|---|---|---|
| *Amblyseius barkeri* | polyphag | Thripse, Weichhautmilben |
| *Amblyseius californicus* | polyphag | Spinnmilben, Thripse |
| *Amblyseius cucumeris* | polyphag | Thripse |
| *Amblyseius swirskii* | polyphag | Weiße Fliege, Thripse, Spinnmilben |
| *Amblyseius degenerans* | polyphag | Thripse |
| *Hypoaspis miles* | polyphag | Trauermücken- und Thrips-Larven, Springschwänze |
| *Hypoaspis aculeifer* | polyphag | Trauermücken-Larven |
| *Phytoseiulus persimilis* | monophag | Gemeine Spinnmilbe |
| *Typhlodromus pyri* | oligophag | Spinnmilben |

Die Erfindung umfasst weiterhin eine Zusammensetzung zur Massenzucht von räuberischen Spinnentieren, wobei die Zusammensetzung ein mikroverkapseltes Nahrungssubstrat umfasst. Bezüglich weiterer Einzelheiten des mikroverkapselten Nahrungssubstrats wird auf die obige Beschreibung verwiesen. Diese Zusammensetzung weist vorzugsweise darüber hinaus wenigstens ein Hilfsmittel bzw. Hilfsmaterialien auf, die insbesondere zur Schaffung von Hohlräumen innerhalb der Zusammensetzung dienen und die Bewegungsfreiheit für die zu züchtenden Spinnentiere und die Möglichkeit für einen ausreichenden Gasaustausch schaffen. Das oder die Hilfsmittel können auch dafür sorgen, dass es nicht zu einem Verklumpen der einzelnen Mikrokapseln kommt, was die Zugänglichkeit der Mikrokapseln für die zu züchtenden Spinnentiere erschweren würde. Geeignete Hilfsmaterialien sind beispielsweise Vermiculit, Buchweizenspelze, Weizenkleie und/oder Hirsespelze.

Die Erfindung umfasst weiterhin ein Verfahren zur Massenzucht von räuberischen Spinnentieren. Hierfür wird eine Zusammensetzung bereitgestellt, die ein mikroverkapseltes Nahrungssubstrat gemäß der obigen Beschreibung und vorzugsweise wenigstens ein Hilfsmittel insbesondere zur Schaffung von Hohlräumen innerhalb der Zusammensetzung umfasst. Der Züchtungsansatz wird mit Individuen oder Eiern der zu züchtenden räuberischen Spinnentiere versetzt. Der Züchtungsansatz wird unter geeigneten Bedingungen, beispielsweise zwischen 18 und 35 °C und zwischen 60 und 95 % relativer Luftfeuchtigkeit gehalten, sodass es zu einer Vermehrung der zu züchtenden Spinnentiere kommt, die das mikroverkapselte Nahrungssubstrat verzehren. Besonders bevorzugt kann die Temperatur zwischen 25 - 28 °C und die relative Luftfeuchtigkeit zwischen 75 - 85 % eingestellt werden.

Weiterhin umfasst die Erfindung ein Präparat für die biologische Schädlingsbekämpfung, das ein mikroverkapseltes Nahrungssubstrat für räuberische Spinnentiere umfasst, wobei die räuberischen Spinnentiere zur Bekämpfung von Pflanzenschädlingen vorgesehen sind. Hierbei handelt es sich insbesondere um Raubmilben wie beispielsweise *Amblyseius swirskii, Phytoseiulus persimilis* oder *Typhlodromus pyri.* Dieses Präparat weist neben dem mikroverkapselten Nahrungssubstrat auch die Spinnentiere selbst sowie vorzugsweise ein Hilfsmittel insbesondere zur Schaffung von Hohlräumen innerhalb des Präparates für die Bewegungsfreiheit der Spinnentiere auf. Bezüglich weiterer Einzelheiten des mikroverkapselten Nahrungssubstrats wird auf die obige Beschreibung verwiesen. Dieses Präparat ist für den Transport der zur biologischen Schädlingsbekämpfung vorgesehenen räuberischen Spinnentiere zum Anwender geeignet. Innerhalb des Präparates werden die räuberischen Spinnentiere mit Nahrung versorgt, sodass es im Wesentlichen nicht zu Qualitätsverlusten der Spinnentiere im Hinblick auf deren Schädlingsbekämpfungseigenschaften während des Transportes kommt. Die Verwendung von einem oder mehreren Hilfsmitteln oder Hilfsmaterialien innerhalb des Präparates schützt das Nahrungssubstrat beispielsweise vor dem Verklumpen und gewährleistet die Zugänglichkeit des Nahrungssubstrats für die Spinnentiere. Weiterhin kann das oder können die Hilfsmittel einen weiteren Schutz für die Spinnentiere während des Transportes, zum Beispiel bei Stößen und Erschütterungen, gegen Überhitzung usw. gewährleisten.

Das Präparat zur biologischen Schädlingsbekämpfung kann als Streuware bereitgestellt werden, wobei das Präparat direkt in die zu behandelnde Pflanzenkultur eingebracht wird. In einer anderen Ausführungsform wird das Präparat als Beutelware bereitgestellt. Hierbei können beispielsweise circa 250 Spinnentiere sich in einem kleinen Beutel befinden, der vom Anwender an die zu behandelnden Pflanzen gehängt wird. Der Beutel weist eine sehr kleine Öffnung auf, durch die die Spinnentiere, beispielsweise die Raubmilben, den Beutel über einen längeren Zeitraum hinweg verlassen. Durch das mikroverkapselte Nahrungssubstrat innerhalb der Beutel können sich die räuberischen Spinnentiere auch noch in den Beuteln vermehren, sodass sie über einen Zeitraum von beispielsweise bis zu zwei Wochen in den Pflanzenbestand einwandern können. Die Beutelware hat daher eine länger andauernde Wirkung als Streuware und eignet sich auch für eine prophylaktische Behandlung von Pflanzenkulturen. Bei der Streuware hingegen werden alle Spinnentiere auf einmal freigesetzt. Hierdurch wird ein schnellerer Anwendungserfolg erzielt und es können höhere Schädlingsdichten erfolgreich bekämpft werden.

Die erfindungsgemäß verwendeten Mikrokapseln können im Prinzip nach bekannten Methoden hergestellt werden, beispielsweise nach dem Vertropfungsverfahren, PGSS-Verfahren (*Partikel from Gas Saturated Solution*) oder dem Rührkesselverfahren. In ganz besonders bevorzugter Weise ist jedoch das nachfolgend beschriebene Verfahren zur Herstellung der Mikrokapseln geeignet, das ebenfalls von der Erfindung umfasst wird. Ähnliche Mikroverkapselungsverfahren sind aus der Literatur bereits bekannt (siehe z.B. Pommersheim et al. in Macromol. Chem. Phys. 195, 1557-1567 (1994*)*). Ausgehend von einem solchen Verkapselungsverfahren wurde ein Verfahren entwickelt, dass die Herstellung von Mikrokapseln erlaubt, die als Futtersubstrat für die Zucht von räuberischen Spinnentieren geeignet sind.

Für dieses Verfahren wird zunächst eine flüssige, insbesondere wässrige Mischung aus dem zu verkapselnden Material, insbesondere einer wässrigen Nährstoff- oder Nährmittellösung, und einem Matrix-bildenden Material bereitgestellt. Bei dem Matrix-bildenden Material handelt es sich insbesondere um Alginat-Komplexe, vorzugsweise um Natrium-Alginat, sodass mit dem erfindungsgemäßen Verfahren Mikrokapseln auf Alginatbasis hergestellt werden. Durch Formgebung dieser Mischung wird die Grundstruktur bzw. Grundform der Mikrokapseln bereitgestellt. Die Formgebung kann beispielsweise als Vertropfung mit Hilfe einer speziellen Düse stattfinden, in der ein konzentrischer Luftstrom Kügelchen bzw. Partikel formt. Durch die Wahl der Düsengröße kann der Durchmesser der Kügelchen vorgegeben werden, beispielsweise in einem Bereich zwischen 100 µm und einigen Millimetern. Die Form der Kügelchen wird in einer lonenlösung, beispielsweise einer CaCl₂-Lösung oder der Lösung eines anderen mehrwertigen Ions, insbesondere eines Metallions, stabilisiert, wobei sich eine gelartige Matrixstruktur ausbildet. Auf diese gelartigen Partikel oder Kügelchen werden Substanzen aufgebracht, die eine Diffusionssperrschicht ausbilden. Hierfür werden Ethylcellulose und/oder Polymere auf Harzbasis, insbesondere Schellack, verwendet. Die Partikel können mit Lösungen der Substanzen umspült oder besprüht werden und anschließend beispielsweise in einem Luftstrom getrocknet werden. Weiterhin können das Aufbringen der Substanzen und das Trocknen auch im Zuge einer Sprühtrocknung zugleich erfolgen. Es können ein oder mehrere Schichten aufgebracht werden, die einen Diffusionsschutz für das Lösungsmittel in dem Inneren der Mikrokapseln bewirken, sodass die für ein Massenzuchtverfahren erforderliche Stabilität bzw. Haltbarkeit der Mikrokapseln gewährleistet ist. Wahlweise können noch weitere Beschichtungen erfolgen, die beispielsweise die Attraktivität der Mikrokapseln für die zu züchtenden Tiere erhöhen. Die Mikrokapseln weisen eine getrocknete Oberfläche auf und können in der Gasphase, d.h. also insbesondere an der Luft, über einen längeren Zeitraum gelagert und verwendet werden.

In einer weiteren bevorzugten Ausgestaltung des Mikroverkapselungsverfahrens werden vor der Ausbildung der Diffusionssperrschicht weitere Substanzen, vorzugsweise natürliche Polymere, auf die Gelpartikel aufgebracht, die insbesondere unter Ausbildung von Polyelektrolyt-Komplexen eine Hüllstruktur bilden. Hierdurch kann zum Einen die Stabilität der Mikrokapseln gesteigert werden und zum Anderen können hierbei Substanzen auf die Kapseln aufgebracht werden, die die Attraktivität der Kapseln für die zu züchtenden Tiere erhöht. Vorzugsweise werden wenigstens ein Polykation, z.B. ein Chitinderivat, insbesondere Chitosan, und wenigstens ein Polyanion, vorzugsweise Pektin, Carboxymethylcellulose und/oder Alginat, verwendet.

Mit besonderem Vorteil können die Mikrokapseln insbesondere nach Ausbildung der Polyelektrolyt-Komplexe einer lonenaustauschbehandlung unterzogen werden, wobei die Viskosität im Inneren der Mikrokapseln erniedrigt wird. Der lonenaustausch kann beispielsweise durch Behandlung mit einer Na-Citrat-Lösung erfolgen. Die Erniedrigung der Viskosität im Kern der Mikrokapseln kann vorteilhaft sein, da hierdurch die Verfügbarkeit des Futtermittels im Inneren der Mikrokapseln, je nach zu züchtendem Tier, verbessert werden kann. Nach dieser sogenannten Rückverflüssigung können, wie bereits beschrieben, die Substanzen zur Ausbildung der Diffusionssperrschicht aufgebracht werden. Die beschriebenen Prozesse können in verschiedenen Bädern oder beispielsweise auch durch Besprühen stattfinden. Die in den Lösungen verwendeten Konzentrationen der Substanzen und die verschiedenen verwendeten Ionen und deren Konzentration beeinflussen die Eigenschaften der Hülle, insbesondere deren Dicke und Festigkeit.

Das beschriebene Herstellungsverfahren für Mikrokapseln ist in besonderer Weise für die Mikroverkapselung von einem Nahrungssubstrat für die Massenzucht von räuberischen Spinnentieren oder anderen nützlichen Organismen, beispielsweise Insekten, geeignet. Das Verfahren ist sehr schonend für die Bestandteile der Nährmittellösung, beispielsweise finden keine Denaturierung von Proteinen oder anderweitige Veränderungen der Komponenten der Nährmittellösung statt, die die Qualität der Nährmittellösung erheblich verschlechtern würde. Für die Verkapselung können ausschließlich natürliche oder naturähnliche Polymere eingesetzt werden, die nicht chemisch verändert werden müssen, sodass sie unbeeinflusst durch das Verkapselungsverfahren für die zu züchtenden Tiere attraktiv sind. Die eingesetzten Substanzen (Ethylcellulose und/oder Schellack und beispielsweise Chitosan und Pektin) bilden eine mehrschichtige stützende und verdunstungssichere Membran aus, die die Hülle für eine Mikrokapsel bildet, die für die zu züchtenden Spinnentiere sehr attraktiv ist, sodass die Mikrokapsel als Nahrungsquelle akzeptiert wird. Der Diffusionsschutz der Mikrokapseln bewirkt eine lange Haltbarkeit und Verwendbarkeit der Mikrokapseln. Vor allem für ein Massenzuchtverfahren ist der Diffusionsschutz aus prozesstechnischen Gründen sehr vorteilhaft. Die Eigenschaften der Hülle der erfindungsgemäß hergestellten Mikrokapseln lassen sich durch Wahl der geeigneten Parameter bei der Herstellung an die natürliche Beute anpassen. So kann die physikalische Struktur der Mikrokapseln, insbesondere die Dicke der Hüllmembran und deren Festigkeit, der natürlichen Beute nachempfunden werden. Schließlich ist das erfindungsgemäße Verkapselungsverfahren sehr gut für eine Massenproduktion geeignet.

Die Erfindung umfasst schließlich auch die erfindungsgemäß herstellbaren Mikrokapseln sowie die Verwendung der Mikrokapseln für die Massenzucht von räuberischen Spinnentieren. Daneben sind die erfindungsgemäßen Mikrokapseln auch für andere Anwendungen geeignet. Zunächst können die Mikrokapseln allgemein als Futtermittel beispielsweise für die Zucht von Insekten oder anderen Organismen eingesetzt werden. Darüber hinaus sind auch andere Anwendungen möglich, beispielsweise in der Biotechnologie, Pharmazie oder bei der Waschmittelherstellung, z.B. zur Verkapselung von Enzymen.

Weitere Merkmale und Vorteile der Erfindung ergeben sich aus der nachfolgenden Beschreibung von Ausführungsbeispielen. Hierbei können die einzelnen Merkmale jeweils für sich oder in Kombination miteinander verwirklicht sein.

### Ausführungsbeispiele

### 1. Amblyseius swirskii

Die polyphage Raubmilbe *Amblyseius swirskii* kann mit sehr guter Wirkung gegen Thripse und gegen die Weiße Fliege eingesetzt werden. Da auch eine Ernährung mit Pollen möglich ist, wird eine Etablierung in blühenden Beständen erleichtert und es kann eine nachhaltige Wirkung erreicht werden. *Amblyseius swirskii* hat sich inzwischen zu einer der am häufigsten eingesetzten Raubmilben in Europa entwickelt. Da die bisher durchgeführte Massenzucht von *Amblyseius swirskii* oftmals auf der Verwendung von Dörrobstmilben (*Carpoglyphus lactis*) als lebenden Futtermilben basiert, können die damit einhergehenden bereits erläuterten Nachteile durch die erfindungsgemäße Verwendung eines mikroverkapselten Nahrungssubstrats für die Produktion vermieden werden.

Abou-Awad et al. (Abou-Awad,-B-A; Reda,-A-S; Elsawi,-S-A., 1991: Effects of artficial and natural diets on the development and reproduction of two phytoseiid mites *Amblyseius gossipi* and *Amblyseius swirskii* (Acari: Phytoseiidae). Insect. Sci. Applic.) konnten zeigen, dass mit einer Diät, bestehend aus Hefe, Milch, Saccharose, Glucose und den Aminosäuren Cystin, Prolin und Arginin, Raubmilben grundsätzlich ernährt werden können. Auf Basis dieses Nährmediums wurden erfindungsgemäß Mikrokapseln hergestellt. Für die Herstellung der Mikrokapseln kann beispielsweise ein Vertropfungsverfahren, ein PGSS-Verfahren (*Partikel from Gas Saturated Solution*) oder ein Rührkesselverfahren eingesetzt werden. Für die Fütterungsversuche mit *Amblyseius swirskii* wurde das Rührkesselverfahren unter Verwendung von Alginat als Hüllmaterial durchgeführt. Zunächst wurden der Wandbildner (Alginat) in einem geeigneten Lösungsmittel gelöst und hierbei Verunreinigungen und unlösliche Bestandteile entfernt. Das Kernmaterial, also die oben genannten Nährsubstanzen, wurden in einem geeigneten Lösungsmittel dispergiert. Die Wandbildung wurde unter Übersättigungssteuerung und Wärmeübertragung durchgeführt und der Ansatz in die Phase, die die Mikrokapseln enthielt, und in die flüssige Phase aufgetrennt. Bei dem anschließenden behutsamen Waschen der Mikrokapseln wurde eine mechanische Schädigung der Mikrokapseln vermieden. Die Trocknung der Mikrokapseln erfolgte ebenfalls behutsam, um harte Agglomerate und eine thermische Schädigung des Materials zu vermeiden. Die Vermessung der Hülle der Mikrokapseln ergab eine Dicke von ca. 20 µm. Der Durchmesser der Kapseln betrug ca. 800 µm.

Die mit diesem Futtermittel erreichte Eiablagerate betrug im Mittel 0,5 Eier pro Weibchen und Tag und war damit vergleichbar mit denen von Abou-Awad et al. (1991) ermittelten Werten. Hingegen legten die Raubmilben, die in einem eigenen durchgeführten Kontrollversuche mit nicht eingekapselter flüssiger Nährlösung gefüttert wurden, keine Eier ab. Durch die erfindungsgemäße Mikroverkapselung des flüssigen Futtermittels konnte ein schnelles Austrocknen der Nährlösung, die 1 x täglich in Form kleiner Tröpfchen auf die Versuchseinheiten gegeben wurden, verhindert werden. Die Ergebnisse deuten zudem darauf hin, dass der Futterreiz für die Raubmilben durch die Mikroverkapselung verstärkt wurde. Die kurze Verfügbarkeit der flüssigen Darreichungsform reichte zwar aus, um die Raubmilben über den Versuchszeitraum am Leben zu halten, der erhöhte Nährstoffbedarf während der Eiablagephase konnte jedoch nicht gedeckt werden. Die Versuche haben gezeigt, dass *Amblyseius swirskii* die Mikrokapseln als Beute akzeptiert und dass das flüssige Futtermittel in einer physiologisch verwertbaren Form im Kapselinneren vorliegt. Der Austrocknungsprozess der Nährlösung wird durch die feste Hülle erheblich verlangsamt. Ein mikroverkapseltes Nahrungssubstrat kann daher mit allen beschriebenen Vorteilen für die Zucht von räuberischen Raubmilben unter Verzicht auf Lebendfutter eingesetzt werden.

Die Ergebnisse lassen sich auch auf andere Raubmilbenarten übertragen. Die notwendigen Anpassungen des flüssigen Nährmediums und der Kapselhülle steigen im Allgemeinen mit dem Spezialisierungsgrad der Raubmilben auf eine bestimmte Beute an. So ist es vorteilhaft, beispielsweise für die Zucht der oligophagen Raubmilbe *Typhlodromus pyri* oder der monophagen Raubmilbe *Phytoseiulus persimilis,* die sich nur von der Gemeinen Spinnmilbe ernähren kann, beutespezifische Strukturen in der Kapselhülle einzufügen, damit das mikroverkapselte Nahrungssubstrat als Beute erkannt und als Futter akzeptiert wird.

### 2. Typhlodromus pyri

Die Raubmilbe *Typhlodromus pyri* ist die wichtigste Raubmilbe im Obst- und Weinbau, wo sie zur Bekämpfung der Spinnmilbe eingesetzt wird. Weiterhin zeigen Versuche im Hopfenanbau, dass Spinnmilbenmassenvermehrungen durch den Einsatz von *Typhlodromus pyri* verhindert werden können. Neben Spinnmilben verzehrt *Typhlodromus pyri* auch Pollen und ist somit als oligophag einzustufen. Durch das Ausweichen auf Pollen ist sie in der Lage, auch bei niedrigem Spinnmilbenbefall im Bestand zu überdauern. Bisher wird *Typhiodromus pyri* über Schnittholz von einer Obst- oder Weinbauanlage zu einer neuen Anlage transportiert. Dies birgt allerdings die Gefahr, dass neben dem Nutzorganismus auch Schädlinge in die neue Anlage eingebracht werden. Bisherige Versuche zur Massenproduktion von *Typhiodromus pyri* sind gescheitert, da kein zufrieden stellendes Produktionssystem gefunden werden konnte, um diese Raubmilbenart in ausreichender Menge zu einem wirtschaftlichen Preis zur Verfügung stellen zu können. Es sind bisher nur sehr geringe Produktionsmengen durch die Verwendung ausschließlich des natürlichen Beuteorganismus möglich. Daneben birgt auch in diesem Fall der Einsatz der natürlichen Beuteorganismen bei der Zucht die Gefahr, dass mit den gezüchteten Raubmilben die zu bekämpfende Spinnmilbe in die Pflanzenkultur zusätzlich eingebracht wird. Zur Produktion dieser Raubmilbe ist das erfindungsgemäße Massenzuchtverfahren mit großem Vorteil einsetzbar.

### 3. Phytoseiulus persimilis

Die Raubmilbe *Phytoseiulus persimilis* ist monophag und kann sich nur von der Spinnmilbe *Tetranychus urticae* (Gemeine Spinnmilbe oder "Rote Spinne") ernähren. *Tetranychus urticae* ist einer der bedeutendsten Schädlinge in Gewächshauskulturen (Tomaten, Paprika, Rosen), aber auch im Freiland (Erdbeeren, Freilandgurken). Sie ist chemisch nur sehr schwer zu bekämpfen und weist zunehmende Resistenzen gegen chemische Pflanzenschutzmittel auf. Die Raubmilbe *Phytoseiulus persimilis* ist ein sehr geeigneter Nützlingsorganismus, um die Gemeine Spinnmilbe als Pflanzenschädling zu bekämpfen. Der Einsatz der Raubmilbe *Phytoseiulus persimilis* zur biologischen Schädlingsbekämpfung in größerem Umfang ist bisher jedoch aufgrund des aufwendigen Massenzuchtverfahrens nicht in wirtschaftlicher Weise möglich, da diese Raubmilbe bislang nur unter Verwendung ihrer natürlichen Beute gezüchtet werden kann. Die Verwendung einer kostengünstiger zu produzierenden Ersatzbeute als Lebendfutter für ein Massenzuchtverfahren ist nicht gelungen. Insbesondere auch für diese Raubmilbe kann das erfindungsgemäße Zuchtverfahren mit besonderem Vorteil eingesetzt werden.

Zur Herstellung der festen Hülle, die bei den Raubmilben einen Fressreiz auslöst, werden Spinnmilben als natürliche Beuteorganismen in Massen produziert, getrocknet und zermahlen. Dieses Pulver enthält die für den Fressreiz benötigten Substanzen und wird auf die feste Hülle aufgebracht bzw. angelagert oder wird bei der Herstellung der festen Hülle in das Hüllmaterial eingemischt. Als Grundsubstanz für das Hüllmaterial werden Biopolymere, beispielsweise Chitosan, Gelatine, Alginat, Pektin oder Mischungen davon eingesetzt, die mit dem Spinnmilbenpulver versetzt werden.

### 4. Verkapselungsverfahren

Vorzugsweise werden die Mikrokapseln für die Zucht der Raubmilben mit einem neuen Verkapselungsverfahren hergestellt, dass zum Einen sehr schonend für die Futtermittelbestandteile ist, zum Zweiten die Hüllenbildung unter Verwendung von natürlichen Polymeren erlaubt und zum Dritten die Aufbringung oder Integration einer Diffusionssperrschicht und gegebenenfalls weiterer Schichten oder Substanzen in einem Herstellungsverfahren ermöglicht, das ohne Weiteres für eine Massenproduktion der Mikrokapseln geeignet ist.

Ein Nährstoffgemisch, das im Hinblick auf seine Zusammensetzung für die Zucht von räuberischen Spinnentieren geeignet ist, wird in eine wässrige 1-2 %-ige Na-Alginat Lösung im Verhältnis von circa 1:1 eingerührt. Das Gemisch wird durch eine geeignete Düse in eine circa 1-2 %ige CaCl₂-Lösung eingetropft. Es bilden sich Gelpartikel, die nach einigen Minuten aus dem Fällbad abgesiebt und in Wasser gewaschen werden.

Die Gelpartikel werden mit einer konzentrierten Schellack-Lösung umspült. Anschließend werden sie abgesiebt und nach einigen Minuten an der Luft mit einer bis zu 10 %-igen Ethylcellulose-Lösung entweder in Lösung oder durch Aufsprühen beschichtet. Danach werden die Partikel in einem Luftstrom getrocknet. Das Aufbringen der Ethylcellulose und/oder des Schellacks sowie das Trocknen der Partikel können auch im Zuge einer Sprühtrocknung gleichzeitig erfolgen.

Wahlweise können die Partikel vor der Beschichtung mit Schellack und/oder Ethylcellulose mehrfach abwechselnd mit verdünnten Lösungen eines Polykations (z.B. Chitosan) und eines Polyanions (z.B. Pektin und/oder Carboxymethylcellulose) umspült werden, wobei sich Membranschichten ausbilden. Anschließend werden die Partikel in Wasser gewaschen und können gegebenenfalls gelagert werden, bevor eine Weiterbehandlung erfolgt. Die auf diese Weise herstellbaren Partikel sind im Prinzip auch als Futtersubstrat für die Zucht von räuberischen Spinnentieren geeignet. Allerdings müssen diese Mikrokapseln in Wasser gelagert werden und sind an der Luft nicht sehr lange haltbar. Vorzugsweise wird daher auf die Partikel mit der Hülle aus Polyelektrolyt-Komplexen eine Diffusionssperrschicht in der oben beschriebenen Weise aufgebracht. Vor dem Aufbringen der Diffusionssperrschicht können die Partikel mit einer verdünnten Na-Citrat-Lösung behandelt werden. Durch den hierbei eintretenden lonenaustausch erniedrigt sich die Viskosität im Inneren der Mikrokapseln. Dies kann für die Verfügbarkeit des Futtermittels für die zu züchtenden Tiere vorteilhaft sein.

Die Kapseln aus mehreren Versuchsreihen wurden Belastungstests unterzogen, um die Festigkeit der Membranhüllen zu ermitteln. Dazu wurde eine Apparatur aus der Materialprüfung eingesetzt, die eine Kapsel mit zunehmender Kraft auf eine Analysenwaage drückt. Gemessen und aufgezeichnet wird das Gewicht, das auf die Waage drückt, in Abhängigkeit vom Abstand zur Auflagefläche. Bei intakter Kapseloberfläche verringert sich mit zunehmendem Druck der Abstand zur Auflagefläche nur sehr langsam - die elastische Kapsel wird verformt. Wird der Kapseldruck jedoch zu hoch, platzt die Membran und der Abstand zur Auflagefläche verringert sich schlagartig gegen Null. Nach Ermittlung der zur Zerstörung erforderlichen Kraft (Zerstörungspunkt) kann bei bekannter Kapseloberfläche die Reißspannung [N/mm²] errechnet werden.

Mit diesem Verfahren wurde ebenfalls die Reißspannung von verschiedenen Beuteorganismen von Raubmilben ermittelt, insbesondere von Spinnmilben, Florfliegeneiern und Milbeneiern. Diese Werte dienen als Referenz für die zu fertigenden Futterkapseln, wobei die Festigkeit der zu fertigenden Mikrokapseln so ausgelegt wurde, dass sie unterhalb der Festigkeit der natürlichen Beuteorganismen liegt, sodass die Raubmilben die Kapselmembranen ohne Weiteres durchstoßen können, um an die Nährlösung im Kern der Kapseln zu gelangen. Die Messungen ergaben für Florfliegeneier die niedrigste Reißspannung, das heißt, dass die Hülle der Florfliegeneier die geringste Festigkeit der getesteten Beuteorganismen zeigt.

Die Membrandicke der gefertigten Kapseln wurde mit Hilfe einer optischen Messeinrichtung an einem Mikroskop gemessen, wobei als Messobjekt Membranen von Kapseln dienten, die durch einen osmotischen Schock zerstört wurden.

Mit diesem Verfahren wurden Mikrokapseln mit einem Durchmesser von circa 0,5 mm und einem Wassergehalt von > 80% hergestellt. Die Membranen der Kapseln wiesen eine Dicke von < 1 µm auf. Die Belastungstests ergaben eine Festigkeit von circa 0,1 N/mm². Dies entspricht etwa der Festigkeit von Florfliegeneiern (0,17 N/mm²), die von Raubmilben gut durchdrungen werden können, bzw. liegt etwas darunter.

## Patentansprüche

1. Verwendung eines mikroverkapselten Nahrungssubstrats zur Massenzucht von räuberischen Spinnentieren, wobei das mikroverkapselte Nahrungssubstrat Mikrokapseln umfasst, die eine feste Hülle aus im Wesentlichen natürlichen Polymeren aufweisen, **dadurch gekennzeichnet, dass** die feste Hülle eine Diffusionssperrschicht umfasst, wobei die Diffusionssperrschicht Ethylcellulose und/oder Polymere auf Harzbasis, insbesondere Schellack umfasst.

2. Verwendung nach Anspruch 1, **dadurch gekennzeichnet, dass** die natürlichen Polymere Polysaccharide, insbesondere Chitin und/oder Derivate von Chitin und/oder Chitosan und/oder Alginat und/oder Carrageen und/oder Pektin und/oder Gummiarabikum und/oder Cellulosederivate, und/oder Proteine, insbesondere Gelatine, und/oder Mischungen davon umfassen.

3. Verwendung nach einem der vorhergehenden Ansprüche, **dadurch gekennzeichnet, dass** die feste Hülle Strukturen und/oder Substanzen aufweist, die für die räuberischen Spinnentiere attraktiv sind.

4. Verwendung nach einem der vorhergehenden Ansprüche, **dadurch gekennzeichnet, dass** die Schichtdicke der festen Hülle im Wesentlichen bis zu 10 µm, vorzugsweise bis zu 5 µm, beträgt, wobei vorzugsweise der Durchmesser der Mikrokapseln 0,1 bis 2 mm beträgt.

5. Verwendung nach einem der vorhergehenden Ansprüche, **dadurch gekennzeichnet, dass** die räuberischen Spinnentiere Milben, insbesondere Raubmilben sind, vorzugsweise Vertreter der Familien Phytoseiidae und Laelapidae, insbesondere die Gattungen Amblyselus, Neoselulus, Hypoaspis, Phytoseiulus oder Typhlodromus, vorzugsweise Amblyseius barkeri, Amblyseius californicus, Amblyseius cucumeris, Amblyseius swirskii, Amblyseius degenerans, Hypoaspis miles, Hypoaspis aculeifer, Phytoseiulus persimilis oder Typhlodromus pyri.

6. Zusammensetzung zur Massenzucht von räuberischen Spinnentieren, umfassend ein mikroverkapseltes Nahrungssubstrat mit den Merkmalen gemäß einem der Ansprüche 1 bis 5 und vorzugsweise wenigstens ein Hilfsmittel.

7. Verfahren zur Massenzucht von räuberischen Spinnentieren, umfassend die folgenden Verfahrensschritte:
- Bereitstellung eines mikroverkapselten Nahrungssubstrats mit den Merkmalen gemäß einem der Ansprüche 1 bis 5 in einem zuchtansatz,
- Bestückung des Zuchtansatzes mit Individuen oder Eiern der räuberischen Spinnentiere und
- Vermehrung der räuberischen Spinnentiere unter geeigneten Zuchtbedingungen.

8. Präparat zur biologischen Schädlingsbekämpfung, umfassend ein mikroverkapseltes Nahrungssubstrat mit den Merkmalen gemäß einem der Ansprüche 1 bis 5 und räuberische Spinnentiere, insbesondere Raubmilben, wobei vorzugsweise das Präparat weiterhin wenigstens ein Hilfsmittel umfasst.

9. Präparat nach Anspruch 8, **dadurch gekennzeichnet, dass** das Präparat als Streuware oder als Beutelware bereitgestellt ist.

10. Verfahren zur Herstellung von Mikrokapseln, umfassend die folgenden Verfahrensschritte:
- Bereitstellen einer flüssigen, insbesondere wässrigen Mischung aus einem zu verkapseinden Material, insbesondere einem Nährmittelgemisch, und einem Matrix-bildenden Material, vorzugsweise Alginat-Komplexen, insbesondere Natrium-Alginat,
- Formgebung der Mischung zur Bereitstellung der Grundstruktur der Mikrokapseln,
- Stabilisierung der Form in einer lonenlösung, insbesondere in einer CaCl₂-Lösung, zur Ausbildung einer gelartigen Matrix, und
- Aufbringen von Substanzen zur Ausbildung einer Diffusionssperrschicht, wobei die Substanzen Ethylcellulose und/oder Polymere auf Harzbasis, insbesondere Schellack, sind.

11. Verfahren nach Anspruch 10, **dadurch gekennzeichnet, dass** vor dem Aufbringen der Substanzen zur Ausbildung der Diffusionssperrschicht weitere Substanzen auf die gelartige Matrix zur Ausbildung einer Hüllstruktur aufgebracht werden, insbesondere wenigstens ein Polykation, vorzugsweise ein Chitinderivat, insbesondere Chitosan, und wenigstens ein Polyanion, vorzugsweise Pektin und/oder Carboxymethylcellulose und/oder Alginat.

12. Verfahren nach Anspruch 11, **dadurch gekennzeichnet, dass** nach dem Aufbringen der weiteren Substanzen zur Ausbildung einer Hüllstruktur die Viskosität im Inneren der Mikrokapsel durch einen lonenaustausch erniedrigt wird, vorzugsweise durch Behandlung mit einer Na-Citrat-Lösung.

13. Mikrokapseln mit einer festen Hülle, wobei die feste Hülle eine Diffusionssperrschicht umfasst und wobei die Diffusionssperrschicht Ethylcellulose und/oder Polymere auf Harzbasis umfasst, **dadurch gekennzeichnet, dass** die Mikrokapseln nach einem Verfahren gemäß einem der Ansprüche 10 bis 12 herstellbar sind.

14. Verwendung von Mikrokapseln gemäß Anspruch 13 zur Massenzucht von räuberischen Spinnentieren.

## Claims

1. Use of a micro-encapsulated food substrate for mass breeding predatory arachnids, wherein the micro-encapsulated food substrate comprises microcapsules that have a solid shell substantially made of natural polymers, **characterized in that** the solid shell comprises a diffusion barrier layer, wherein the diffusion barrier layer comprises ethyl cellulose and/or polymers based on resin, in particular shellac.

2. Use according to claim 1, **characterized in that** the natural polymers include polysaccharides, in particular chitin and/or derivatives of chitin and/or chitosan and/or alginate and/or carrageenan and/or pectin and/or acacia gum and/or cellulose derivatives, and/or proteins, in particular gelatin, and/or mixtures thereof.

3. Use according to any one of the preceding claims, **characterized in that** the solid shell comprises structures and/or substances that are attractive to the predatory arachnids.

4. Use according to any one of the preceding claims, **characterized in that** the layer thickness of the solid shell is essentially up to 10 µm, preferably up to 5 µm, wherein the diameters of the microcapsules are preferably 0.1 to 2 mm.

5. Use according to any one of the preceding claims, **characterized in that** the predatory arachnids are mites, in particular predatory mites, preferably representatives from the families consisting of Phytoseiidae and Laelapidae, in particular the genera Amblyseius, Neoseiulus, Hypoaspis, Phytoseiulus or Typhlodromus, preferably Amblyseius barkeri, Amblyseius californicus, Amblyseius cucumeris, Amblyseius swirskii, Amblyseius degenerans, Hypoaspis miles, Hypoaspis aculeifer, Phytoseiulus persimilis or Typhlodromus pyri.

6. A composition for mass breeding predatory arachnids, comprising a micro-encapsulated food substrate having the characteristics according to any one of claims 1 to 5 and preferably at least one auxiliary agent.

7. A method for mass breeding predatory arachnids, comprising the following steps:
- providing a micro-encapsulated food substrate having the characteristics according to any one of claims 1 to 5 in a breeding starter culture;
- populating the breeding starter culture with individuals or eggs of the predatory arachnids; and
- reproducing the predatory arachnids under suitable breeding conditions.

8. A preparation for biological pest control, comprising a micro-encapsulated food substrate having the characteristics according to any one of claims 1 to 5 and predatory arachnids, in particular predatory mites, wherein the preparation preferably further comprises at least one auxiliary agent.

9. The preparation according to claim 8, **characterized in that** the preparation is provided as loose product or bagged product.

10. Method for producing microcapsules, comprising the following steps:
- providing a liquid, in particular an aqueous, mixture of a material to be encapsulated, in particular a nutriment mixture, and a matrix-forming material, preferably alginate complexes, in particular sodium alginate;
- shaping the mixture so as to provide the basic structure of the microcapsules;
- stabilizing the shape in an ion solution, in particular in a CaCl₂ solution, so as to form a gel-like matrix; and
- applying substances so as to create a diffusion barrier layer, wherein the substances are ethyl cellulose and/or polymers on resin basis, in particular shellac.

11. The method according to claim 10, **characterized in that**, before the substances for creating the diffusion barrier layer are applied, additional substances are applied to the gel-like matrix so as to form a shell structure, in particular at least one polycation, preferably a chitin derivative, in particular chitosan, and at least one polyanion, preferably pectin and/or carboxymethyl cellulose and/or alginate.

12. The method according to claim 11, **characterized in that**, after the additional substances for creating a shell structure are applied, the viscosity in the interior of the microcapsule is reduced by way of ion exchange, preferably by treatment with a Na citrate solution.

13. Microcapsules comprising a solid shell, wherein the solid shell comprises a diffusion barrier layer, and wherein the diffusion barrier layer comprises ethyl cellulose and/or polymers on resin basis, **characterized in that** the microcapsules can be produced by a method according to any one of claims 10 to 12.

14. Use of microcapsules according to claim 13 for mass breeding predatory arachnids.

## Revendications

1. Utilisation d'un substrat alimentaire micro-encapsulé pour l'élevage de masse d'arachnides prédateurs, dans laquelle le substrat alimentaire micro-encapsulé comprend des microcapsules comportant une enveloppe solide constituée essentiellement de polymères naturels, **caractérisée en ce que** l'enveloppe solide comprend une couche barrière de diffusion, dans laquelle la couche barrière de diffusion comprend de la cellulose éthylique et/ou des polymères à base de résine, en particulier de la gomme laque.

2. Utilisation selon la revendication 1, **caractérisée en ce que** les polymères naturels comprennent des polysaccharides, en particulier de la chitine et/ou des dérivés de chitine et/ou du chitosan et/ou de l'alginate et/ou du carragène et/ou de la pectine et/ou de la gomme arabique et/ou des dérivés de cellulose, et/ou des protéines, en particulier de la gélatine, et/ou des mélanges de ceux-ci.

3. Utilisation selon l'une des revendications précédentes, **caractérisée en ce que** l'enveloppe solide comporte des structures et/ou des substances capables d'attirer les arachnides prédateurs.

4. Utilisation selon l'une des revendications précédentes, **caractérisée en ce que** l'épaisseur de couche de l'enveloppe solide mesure généralement jusqu'à 10 µm, de préférence jusqu'à 5 µm, le diamètre des microcapsules mesurant de préférence 0,1 à 2 mm.

5. Utilisation selon l'une des revendications précédentes, **caractérisée en ce que** les arachnides prédateurs sont des acariens, en particulier des acariens prédateurs, de préférence des représentants de la famille des Phytoseiidae et des Laelapidae, en particulier les genres des Amblyseius, des Neoselulus, des Hypoaspis, des Phytoseiulus ou des Typhlodromus, de préférence le Amblyseius barkeri, le Amblyseius californicus, le Amblyseius cucumeris, le Amblyseius swirskii, le Amblyseius degenerans, le Hypoaspis miles, le Hypoaspis aculeifer, le Phytoseiulus persimilis ou le Typhlodromus pyri.

6. Composition pour un élevage de masse d'arachnides prédateurs, comprenant un substrat alimentaire micro-encapsulé avec les caractéristiques selon l'une des revendications 1 à 5 et de préférence au moins un adjuvant.

7. Procédé pour l'élevage de masse d'arachnides prédateurs, comprenant les étapes de procédé suivantes :
- mise à disposition d'un substrat alimentaire micro-encapsulé avec les caractéristiques selon l'une des revendications 1 à 5 dans un début d'élevage,
- équipement du début d'élevage avec des individus ou des oeufs d'arachnides prédateurs, et
- multiplication des arachnides prédateurs dans des conditions d'élevage appropriées.

8. Préparation pour la destruction biologique des parasites, comprenant un substrat alimentaire micro-encapsulé avec les caractéristiques selon l'une des revendications 1 à 5 et des arachnides prédateurs, en particulier des acariens prédateurs, dans laquelle la préparation comprend de préférence en outre au moins un adjuvant.

9. Préparation selon la revendication 8, **caractérisée en ce que** la préparation est disponible sous la forme de produit à répandre ou sous la forme de produit en sachet.

10. Procédé pour la fabrication de microcapsules, comprenant les étapes de procédé suivantes :
- mise à disposition d'un mélange liquide, en particulier aqueux, à partir d'un matériau à encapsuler, en particulier un mélange alimentaire, et d'un matériau formant une matrice, de préférence des complexes d'alginate, en particulier de l'alginate de sodium,
- mise en forme du mélange en vue de la préparation de la structure de base des microcapsules,
- stabilisation de la forme dans une solution ionique, en particulier une solution CaCl₂, pour la formation d'une matrice gélatineuse, et
- application de substances pour la formation d'une couche barrière de diffusion, les substances étant de la cellulose éthylique et/ou des polymères à base de résine, en particulier de la gomme laque.

11. Procédé selon la revendication 10, **caractérisé en ce qu'**avant l'application des substances pour la formation de la couche barrière de diffusion, d'autres substances sont appliquées sur la matrice gélatineuse pour former une structure d'enveloppe, en particulier au moins un polycation, de préférence un dérivé de chitine, en particulier du chitosan, et au moins un polyanion, de préférence de la pectine et/ou de la carboxyméthylcellulose et/ou de l'alginate.

12. Procédé selon la revendication 11, **caractérisé en ce que** suite à l'application des autres substances pour la formation d'une structure d'enveloppe, la viscosité à l'intérieur des microcapsules est diminuée par un échange ionique, de préférence par un traitement avec une solution Na-Citrate.

13. Microcapsules avec une enveloppe solide, dans lesquelles l'enveloppe solide comprend une couche barrière de diffusion et dans lesquelles la couche barrière de diffusion comprend de la cellulose éthylique et/ou des polymères à base de résine, **caractérisées en ce que** les microcapsules peuvent être produites à l'aide d'un procédé selon l'une des revendications 10 à 12.

14. Utilisation de microcapsules selon la revendication 13 pour l'élevage de masse d'arachnides prédateurs.
